**Europäisches Patentamt**

**European Patent Office**

**Office européen des brevets**

(19)

(11) Veröffentlichungsnummer : **0 159 610**
**B1**

# (12) EUROPÄISCHE PATENTSCHRIFT

(45) Veröffentlichungstag der Patentschrift :
**02.03.88**

(21) Anmeldenummer : **85104239.0**

(22) Anmeldetag : **09.04.85**

(51) Int. Cl.⁴ : **C 10 J   3/57, C 07 C   29/15,**
**F 02 C   3/28, F 01 K 23/06**

(54) **Gasturbinen- und Dampfkraftwerk mit einer integrierten Kohlevergasungsanlage.**

(30) Priorität : **21.04.84 DE 3415224**

(43) Veröffentlichungstag der Anmeldung :
**30.10.85 Patentblatt 85/44**

(45) Bekanntmachung des Hinweises auf die Patenterteilung : **02.03.88 Patentblatt 88/09**

(84) Benannte Vertragsstaaten :
**BE DE FR GB IT NL SE**

(56) Entgegenhaltungen :
**DE-A- 1 915 248**
**DE-A- 2 425 939**
**DE-A- 2 837 988**
**DE-A- 2 908 748**
**GB-A- 2 029 855**
**GB-A- 2 075 124**
**GB-A- 2 086 389**

(73) Patentinhaber : **Siemens Aktiengesellschaft Berlin**
**und München**
**Wittelsbacherplatz 2**
**D-8000 München 2 (DE)**

(72) Erfinder : **Frewer, Hans, Dr. Dipl.-Ing.**
**Atzelsberger Strasse 25**
**D-8525 Marloffstein (DE)**
Erfinder : **Müller, Rainer, Dr. Dipl.-Ing.**
**herdegenplatz 1**
**D-8520 Erlangen (DE)**
Erfinder : **Schiffers, Ulrich, Dr. Dipl.-Ing.**
**Moritzbergstrasse 1**
**D-8501 Eckental (DE)**

Anmerkung : Innerhalb von neun Monaten nach der Bekanntmachung des Hinweises auf die Erteilung des
europäischen Patents im Europäischen Patentblatt kann jedermann beim Europäischen Patentamt gegen das erteilte
europäische Patent Einspruch einlegen. Der Einspruch ist schriftlich einzureichen und zu begründen. Er gilt erst als
eingelegt, wenn die Einspruchsgebühr entrichtet worden ist (Art. 99(1) Europäisches Patentübereinkommen).

## Beschreibung

Die Erfindung bezieht sich auf ein Gasturbinen- und Dampfkraftwerk mit einer über eine Luftzerlegungsanlage mit Sauerstoff versorgten, integrierten Kohlevergasungsanlage, mit einer an der Kohlevergasungsanlage angeschlossenen Methanolsyntheseanlage, mit einem an den Abhitzekessel der Gasturbine angeschlossenen Dampfkraftwerksteil sowie mit einer von der Luftzerlegungsanlage zur Brennkammer der Gasturbine führenden Stickstoffleitung.

Ein solches Gasturbinen- und Dampfkraftwerk ist bereits vorgeschlagen worden (amtliches Aktenzeichen P 33 19 732.6). Es zeichnet sich durch eine außerordentliche Flexibilität bei der Erzeugung elektrischer Energie aus. Wegen der in Schwachlastzeiten vermehrten Erzeugung von Methanol kann der Kohlevergaser bei diesem Gasturbinen- und Dampfkraftwerk stets mit gleichbleibender, seinem optimalen Wirkungsgrad angepaßter Leistung betrieben werden. Bei dieser vorgeschlagenen Kraftwerkskonzeption werden für die Entfernung des Schwefels eine Schwefelwasserstoffabsorbtionsanlage und eine Clausanlage benötigt.

Der Erfindung liegt die Aufgabe zugrunde, eine Variante dieses Kraftwerkskonzepts zu finden, die preiswerter herstellbar ist und bei der die Abgase ebenfalls so gut wie schwefelfrei sind.

Die gestellte Aufgabe wird erfindungsgemäß durch die im Kennzeichen des Anspruchs 1 angegebenen Merkmale gelöst. Vorteilhafte Weiterbildungen sind in den Ansprüchen 2 bis 8 beschrieben.

Durch die hydrierende Kohlevergasung und die anschliessende Vergasung des schwefelhaltigen Restkokses in einem Eisenbadvergaser kann der gesamte im fossilen Brennstoff enthaltende Schwefel ohne Zuhilfenahme weiterer Bausteine, durch bloße Zugabe von Kalk in der auf dem Eisenbad schwimmenden Schlacke eingebunden werden. Diese wird dann von Zeit zu Zeit abgelassen. Zugleich können durch die hydrierende Kohlevergasung in beachtlichem Maße Methan und in geringem Maße auch höherwertige Kohlenwasserstoffe erzeugt werden.

Neben der Einbindung des Schwefels erfüllt der Eisenbadvergaser auch die Aufgabe das für die Wasserstofferzeugung benötigte Kohlenmonoxydgas in ausreichender Menge bereitzustellen. Aus diesem kann in der gasseitig nachgeschalteten Konvertierungsanlage Wasserstoffgas und Kohlendioxydgas hergestellt werden. Das Kohlendioxydgas läßt sich in der angeschlossenen Kohlendioxydwaschanlage auswaschen. Weil aber für die Methanolsynthese ein Mengenverhältnis von Kohlenmonoxyd zu Wasserstoff wie 1 : 2 benötigt wird, braucht die Konvertierungsanlage und die Kohlendioxydwaschanlage nur für einen Teil der gesamten Abgasmenge des Eisenbadvergasers ausgelegt zu werden. Dies führt zu einer Verkleinerung der Konvertierungsanlage und der Kohlendioxydwaschanlage. Weil bei der Methanolsynthese auch Kohlendioxyd mit Wasserstoff in Methanol umgewandelt werden können, kann das in der Kohlendioxydwaschanlage ausgewaschene Kohlendioxyd dem in der die Methanolsyntheseanlage zuströmenden Kohlenmonoxydgas beigemischt werden. Auf diese Weise kann der Bedarf an Kohlenmonoxyd vermindert werden, so daß größere Mengen für die Wasserstofferzeugung verfügbar werden. Bei dieser Kraftwerkskonzeption wird die sonst erforderliche Rezirkulation des Wasserstoffgases in den hydrierenden Kohlevergaser mit der Tieftemperaturgaszerlegungsanlage, der Wiederaufwärmung des Wasserstoffgases und der Kompressor für das Wasserstoffgas eingespart.

Infolge der Verwendung einer Durchlaufmethanolsyntheseanlage statt einer solchen mit Rezirkulation von Synthesegas, ist eine exakte Einhaltung des stöchiometrischen Verhältnisses von Kohlenmonoxydgas und Wasserstoffgas nicht erforderlich. Hierdurch können die sonst erforderlichen Meß- und Regeleinrichtungen vereinfacht bzw. weniger aufwendig sein. Auch wird so der Kompressor für das rezirkulierende Synthesegas eingespart und braucht das Kohlenmonoxydgas nicht in dem sonst erforderlichen Maße konvertiert zu werden. Außerdem wird so eine größere Flexibilität beim Betrieb, insbesondere hinsichtlich der Ausnutzung der Rohstoffe in Kohle- und Eisenbadvergaser, und der Art der verwendeten fossilen Brennstoffe erreicht.

Diese heißen Abgase des Eisenbadvergasers werden in einem diesem unmittelbar nachgeschalteten Hochdruckdampferzeuger und -überhitzer abgekühlt. Der dabei erzeugte Hochdruckdampf kann unmittelbar einer Dampfturbine zugeleitet werden. Da er das gleiche Druck- und Temperaturnievau hat wie der im Abhitzekessel der Gasturbine erzeugte Dampf, kann er diesem auch zugemischt werden.

Weitere Flexibilität beim Betrieb dieses Kraftwerks wird dadurch erreicht, daß ein Teil der den Hochdruckdampferzeuger verlassenden Abgase des Eisenbadvergasers über eine separate Leitung zusammen mit den nicht umgesetzten Synthesegasen der Durchlaufmethanolsyntheseanlage der Brennkammer der Gasturbine zuführbar sind. Das hat zur Folge, daß der Gasturbine in Spitzenlastzeiten kurzfristig vermehrt Brennstoff zugeführt und dabei gleichzeitig der Dampfverbrauch für die geringere in die Konvertierungsanlage eingespeiste kohlenmonoxydhaltige Gasmenge vermindert werden kann.

Schließlich kann bei diesem Kraftwerk die Emission von Stickoxyden auch noch dadurch gesenkt werden, daß der in der dem Eisenbadvergaser nachgeschalteten Luftzerlegungsanlage vom Sauerstoff abgetrennte Stickstoff dem Brenner der Gasturbine leistungsbezogen in abgestimmter Menge zugeführt wird. Dadurch läßt sich die Flammtemperatur unter den für die Stickoxydbildung erforderlichen Temperaturniveau halten.

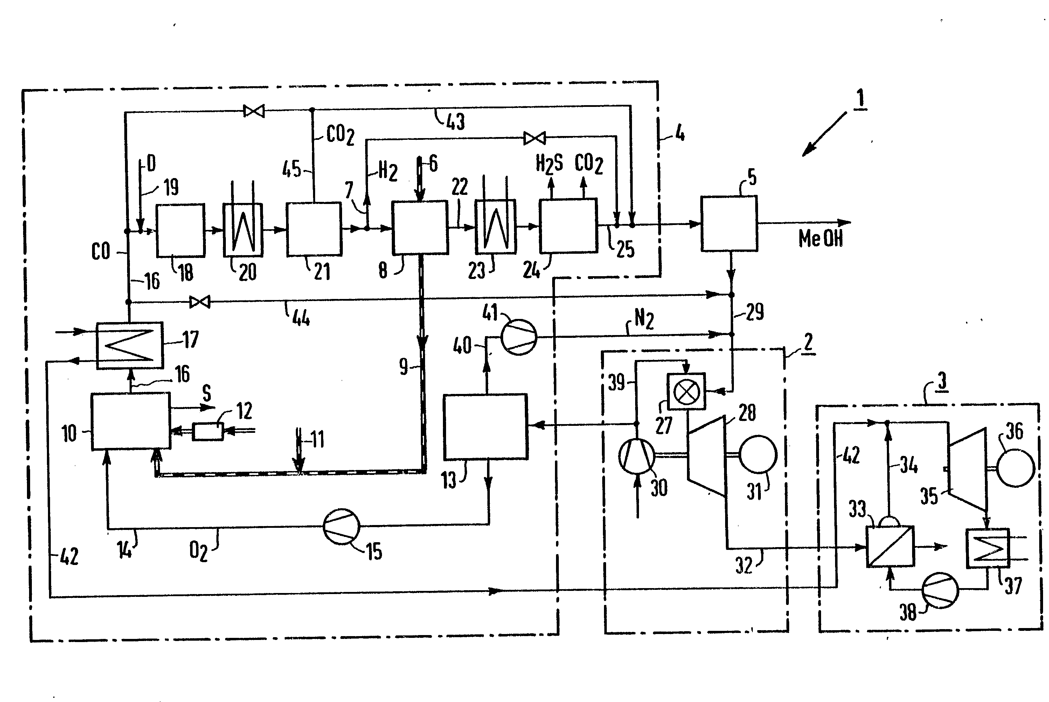

Weitere Einzelheiten der Erfindung werden anhand eines in der Figur dargestellten Ausführungsbeispiels erläutert. Es zeigt :

die Figur eine schematische Darstellung des Aufbaus eines erfindungsgemäßen Gasturbinen-Dampfkraftwerks.

Wie die Figur zeigt, besteht das erfindungsgemäße Gasturbinen- und Dampfkraftwerk 1 aus einem Gasturbinenkraftwerksteil 2, einem Dampfturbinenkraftwerksteil 3 und einer dem Gasturbinenkraftwerksteil vorgeschalteten Anlage 4 zur Kohlevergasung mit einer angeschlossenen Anlage 5 zur Erstellung von Chemierohstoffen, im Ausführungsbeispiel einer Durchlaufmethanolsyntheseanlage. Die zu vergasenden fossilen Brennstoffe, im Ausführungsbeispiel Kohle, gelangt über die Kohleleitung 6 in einem zur hydrierenden Kohlevergasung über eine Wasserstoffversorgungsleitung 7 mit Wasserstoff gespeisten Kohlevergaser 8. Der im Kohlevergaser 8 nicht umgesetzte Restkoks wird über eine Koksleitung 9 in einen Eisenbadvergaser 10 geleitet. In diese Koksleitung 9 mündet außerdem eine weitere Kohleleitung 11, über die zusätzliche fossile Brennstoffe zugeführt werden können. Außerdem ist der Eisenbadvergaser 10 mit einer Dosiereinrichtung 12 für die Zuführung von Kalk versehen. Der Eisenbadvergaser 10 ist an einer mit einer Luftzerlegungsanlage 13 verbundeten Sauerstoffleitung 14 angeschlossen. In dieser ist ein Verdichter 15 zur Überwindung der Druckdifferenz eingebaut.

Im Eisenbadvergaser 10 wird der Brennstoff zu einem vorwiegend kohlenmonoxydhaltigen Gas umgesetzt. An die Abgasleitung 16 des Eisenbadvergasers ist eine Wärmetauscheranlage 17 mit einem Hochdruckdampferzeuger angeschlossen. In die die Wärmetauscheranlage verlassende Abgasleitung 16 des Eisenbadvergasers 10 mündet vor ihrem Anschluß an eine Konvertierungsanlage 18 eine Mitteldruckdampfleitung 19. Dieser Konvertierungsanlage 18, in der das Kohlenmonoxydgas zusammen mit dem beigemischten Wasserdampf zu Wasserstoff und Kohlendioxyd umgesetzt wird, sind gasseitig ein weiterer Wärmetauscher 20 sowie daran anschließend eine Kohlendioxydwaschanlage 21, in der das Kohlendioxyd ausgewaschen wird, nachgeschaltet. Das die Kohlendioxydwaschanlage verlassende, im wesentlichen wasserstoffhaltige Gas, wird in die Wasserstoffversorgungsleitung 7 eingespeist. An diese Wasserstoffversorgungsleitung ist unter Anderem der hydrierende Kohlevergaser 8, in dem die eingesetzten fossilen Brennstoffe zu einem methanreichen Gas umgesetzt werden, angeschlossen.

Die Abgasleitung 22 des hydrierenden Kohlevergasers 8 ist über eine Wärmetauscheranlage 23 an eine aus einer Entstaubungsanlage und einer Kohlendioxyd- und Schwefelwasserstoffwaschanlage bestehenden Gasreinigungsanlage 24 angeschlossen. Deren Synthesegasleitung 25 ist an die eine Durchlaufmethanolsyntheseanlage 5 angeschlossen. In diese mündet auch die Wasserstoffversorgungsleitung 7. Die Durchlaufmethanolsyntheseanlage ist abgasseitig an eine zur Brennkammer 27 der Gasturbine 28 führende Brennstoffleitung 29 angeschlossen.

Die Gasturbine 28 des Gasturbinenkraftwerksteils 2 treibt sowohl ein Luftverdichter 30 für die Verbrennungsluft als auch einen Generator 31 an. An die Abgasleitung 32 der Gasturbine 28 ist ein Abhitzekessel 33 angeschlossen. An die Hochdruck-Dampfleitung 34 des Abhitzekessels 33 ist die Dampfturbine 35 des Dampfturbinenkraftwerksteils 3 angeschlossen. Sie ist mit einem Generator 36 gekuppelt. An der Dampfaustrittsseite der Dampfturbine schließt sich ein Kondensator 37, eine Kondensatpumpe (nicht dargestellt), ein Speisewasserbehälter (nicht dargestellt) und mindestens eine an den Abhitzekessel 33 angeschlossenen Speisewasserpumpe 38 an.

Die Druckluftleitung 39 des Luftverdichters 30 ist einerseits mit der Brennkammer 27 der Gasturbine 28 und andererseits mit der Luftzerlegungsanlage 13 verbunden. Die Sauerstoffleitung 14 der Luftzerlegungsanlage 13 ist über den Verdichter 15 mit dem Eisenbadvergaser 10 und die Stickstoffleitung 40 der Luftzerlegungsanlage 13 über einen weiteren Verdichter 41 mit der zur Brennkammer 27 führenden Brennstoffleitung 29 verbunden. In die vom Abhitzekessel 33 zur Dampfturbine führende Hochdruckdampfleitung 34 mündet die Hochdruckdampfleitung 42 der dem Eisenbadvergaser 10 gasseitig nachgeschalteten Wärmetauscheranlage 17. In die zur Durchlaufmethanolsyntheseanlage 5 führende Synthesegasleitung 25 mündet nicht nur die Wasserstoffversorgungsleitung 7, sondern außerdem auch eine hinter der dem Eisenbadvergaser 10 nachgeschalteten Wärmetauscheranlage 17 abzweigende zweite Abgasleitung 43 für das kohlenmonoxydhaltige Abgas des Eisenbadvergasers. Schließlich zweigt von derselben Stelle noch eine dritte Abgasleitung 44 ab, die direkt in die zur Brennkammer 27 der Gasturbine führende Brennstoffleitung 29 einmündet. Die Abgasleitung 45 für das in der Kohlendioxydwaschanlage 21 abgetrennte Kohlendioxyd ist an die zur Methanolsyntheseanlage 5 führende Abgasleitung 43 für das kohlenmonoxydhaltige Abgas angeschlossen.

Die in den Kohlevergaser 8 eingesetzten fossilen Brennstoffe werden mit Hilfe des aus der Wasserstoffversorgungsleitung 7 entnommenen Wasserstoffgas zu einem im wesentlichen wasserstoff- und methanhaltigen Gas vergast. Der verbleibende Restkoks wird über die Koksleitung 9 gegebenenfalls unter Zumischung weiterer fossiler Brennstoffe in den Eisenbadvergaser 10 eingespeist und dort mit Hilfe des der Luftzerlegungsanlage entnommenen Sauerstoffs zu einem im wesentlichen kohlenmonoxydhaltigen Gas vergast. Zur Einbindung des im Restkoks und in den gegebenenfalls zusätzlich eingeführanderen fossilen Brennstoffen befindlichen Schwefels wird dem Eisenbadvergaser über eine separate Dosiereinrichtung 12 Kalk zugegeben. In der sich so auf dem Eisenbad bildenden Schlacke kann der gesamte im Brennstoff enthaltene Schwefel eingebunden und von Zeit zu Zeit abgelassen werden.

Das den Eisenbadvergaser verlassende sehr heiße, im wesentlichen kohlenmonoxydhaltige, Abgas wird in einer Wärmetauscheranlage 17 abgekühlt und über die Abgasleitung 16 in eine Konvertierungsanlage 18 geleitet. Vor Einmündung in der Konvertierungsanlage wird diesem kohlenmonoxydhaltigen Gas über eine Mitteldruckdampfleitung 19 Wasserdampf zugeführt, so daß das Kohlenmonoxydgas zusammen mit dem Wasserdampf in der Konvertierungsanlage zu Wasserstoff und Kohlenmonoxyd umgesetzt werden kann. Das die Konvergierungsanlage verlassende Gas wird nach Abkühlung in einem Wärmetauscher in eine Kohlendioxydwaschanlage 21 eingeleitet, in der es vom Kohlendioxyd befreit wird. Das die Kohlendioxydwaschanlage verlassende nunmehr im wesentlichen wasserstoffhaltige Gas wird in die Wasserstoffversorgungsleitung 7 eingespeist.

Das den Kohlevergaser 8 verlassende, im wesentlichen Methan sowie Wasserstoff enthaltende Abgas wird in der Wärmetauscheranlage 23 gekühlt und in der Gasreinigungsanlage 24 von mitgerissenen Ascheteilchen sowie von Schwefelwasserstoff und Kohlendioxydresten gereinigt. Es wird sodann über die Synthesegasleitung 25 der Methanoldurchlaufsyntheseanlage zugeleitet. An diese Synthesegasleitung ist sowohl die Wasserstoffversorgungsleitung 7 als auch eine zweite Abgasleitung 43 für nicht konvertiertes kohlenmonoxydhaltiges Abgas des Eisenbadvergasers angeschlossen. Entsprechend dem Verhältnis des durch diese zweite Abgasleitung 43 und durch die Konvertierungsanlage 18 geleiteten Anteils an kohlenmonoxydhaltigen Abgas des Eisenbadvergasers kann das stöchiometrische Verhältnis für die nachgeschaltete Durchlaufmethanolsyntheseanlage von Wasserstoff zu Kohlenmonoxyd wie 2 :1 eingestellt werden. Das die Durchlaufsyntheseanlage verlassende, nicht zu Methanol umgesetzte Syntheseabgas, in dem auch das die Gasreinigungsanlage passierende Methan enthalten ist, gelangt über die Brennstoffleitung in die Brennkammer 27 des Gasturbinenkraftwerksteils.

Infolge der Verbrennung der nicht umgesetzten Syntheserestgase kann eine Anlage zur Entfernung des Methans aus dem Abgas des hydrierenden Kohlevergasers entfallen und sind die Anforderungen an die Einhaltung des stöchiometrischen Verhältnisses von Kohlenmonoxyd und Wasserstoffgas in dem Synthesegas stark vermindert. Seine Einstellung ist darüber hinaus durch Regulierung des Gasdurchflusses in der zweiten Abgasleitung 43 des Eisenbadvergasers 10 einfach.

Diese Konzeption des Gasturbinendampfkraftwerks 1 erlaubt es auch in der Anlaufphase und bei plötzlichen Laständerungen nicht konvertiertes, kohlenmonoxydhaltiges Abgas des Eisenbadvergasers über die dritte Abgasleitung 44 direkt in die zur Brennkammer 27 der Gasturbine 28 führende Brennstoffleitung 29 einzuleiten. Hierdurch kann die Leistung der Gasturbine kurzfristig verändert und mit geringer Verzögerung auch die am Abhitzekessel erzeugte Dampfmenge für die Dampfturbine 35 erhöht werden. Zugleich braucht so durch die verminderte zu konvertierende Abgasmenge auch weniger Mitteldruckdampf für die Konvertierung abgezweigt zu werden. Hierdurch wird die Flexibilität des Kraftwerkes bei der Anpassung seiner elektrischen Leistung an die Anforderungen des Netzes erhöht, ohne daß der hydrierende Kohlevergaser oder der Eisenbadvergaser in seiner Leistung nennenswert verändert zu werden braucht.

Durch die zusätzliche Einspeisung von Stickstoff in die Brennstoffleitung 29 der Brennkammer 27 läßt sich darüber hinaus die Flammtemperatur so weit absenken, daß die Stickoxydemission bei gegebener Leistung minimiert werden kann.

Bezugszeichenliste

| | |
|---|---|
| Gasturbinen- und Dampfkraftwerk | 1 |
| Gasturbinenkraftwerksteil | 2 |
| Dampfturbinenkraftwerksteil | 3 |
| Anlage zur Kohlevergasung | 4 |
| Anlage zur Herstellung von Chemierohstoff (-Methanolsyntheseanlage) | 5 |
| Kohleleitung | 6 |
| Wasserstoffversorgungsleitung | 7 |
| Kohlevergaser | 8 |
| Koksleitung | 9 |
| Eisenbadvergaser | 10 |
| Kohleleitung | 11 |
| Dosiereinrichtung | 12 |
| Luftzerlegungsanlage | 13 |
| Sauerstoffleitung | 14 |
| Verdichter | 15 |
| Abgasleitung (Eisenbadvergaser) | 16 |
| Wärmetauscheranlage | 17 |
| Konvertierungsanlage | 18 |
| Mitteldruckdampfleitung | 19 |
| Wärmetauscher | 20 |
| Kohlendioxydwaschanlage | 21 |
| Abgasleitung (Hydr. Kohlevergaser) | 22 |
| Wärmetauscheranlage | 23 |
| Gasreinigungsanlage | 24 |
| Synthesegasleitung | 25 |
| Brennkammer | 27 |
| Gasturbine | 28 |
| Brennstoffleitung | 29 |
| Luftverdichter | 30 |
| Generator | 31 |
| Abgasleitung (Gasturbine) | 32 |
| Abhitzekessel | 33 |
| HD-Dampfleitung | 34 |
| Dampfturbine | 35 |
| Generator | 36 |
| Kondensator | 37 |
| Speisewasserpumpe | 38 |
| Druckluftleitung | 39 |
| Stickstoffleitung | 40 |
| Verdichter | 41 |
| HD-Dampfleitung | 42 |
| zweite Abgasleitung | 43 |
| dritte Abgasleitung | 44 |
| Dampf | D |
| Methanol | MeOH |
| $CO_2$ Abgasleitung | 45 |

Schlacke S

**Patentansprüche**

1. Gasturbinen- und Dampfkraftwerk mit einer, über eine Luftzerlegungsanlage mit Sauerstoff versorgte integrierte Kohlevergasungsanlage, mit einer an der Kohlevergasungsanlage angeschlossenen Methanolsyntheseanlage, mit einem an den Abhitzekessel der Gasturbine angeschlossenen Dampfkraftwerksteil sowie mit einer von einer Luftzerlegungsanlage zur Brennkammer der Gasturbine führenden Stickstoffleitung, dadurch gekennzeichnet, daß der Kohlevergaser (8) zur hydrierenden Kohlevergasung an eine Wasserstoffversorgungsleitung (7) angeschlossen ist, dem Kohlevergaser zur Verwertung des Restkokses ein Eisenbadvergaser (10) nachgeschaltet ist, dem Abgasseitig eine Konvertierungsanlage (18) und eine an die Wasserstoffversorgungsleitung angeschlossene Kohlendioxyd-Waschanlage (21) nachgeschaltet sind und die Abgasleitung (22) des Kohlevergasers über eine Gasreinigungsanlage (24) an eine Methanolsyntheseanlage (5) und die Abgasleitung der Methanolsyntheseanlage (5) an die Brennkammer (27) der Gasturbine (28) angeschlossen ist.

2. Gasturbinen- und Dampfkraftwerk nach Anspruch 1, dadurch gekennzeichnet, daß dem Eisenbadvergaser (10) außer dem Restkoks des Kohlevergasers (8) zusätzliche fossile Brennstoffe zuführbar sind.

3. Gasturbinen- und Dampfkraftwerk nach Anspruch 1, dadurch gekennzeichnet, daß die Abgasleitung (16) des Eisenbadvergasers durch eine dampfseitig an die Dampfturbine (35) des Dampfkraftwerkteils (3) angeschlossene Wärmetauscheranlage (17) geführt ist.

4. Gasturbinen- und Dampfkraftwerk nach Anspruch 1, dadurch gekennzeichnet, daß die Wasserstoffversorgungsleitung (7) außer an den Kohlevergaser (8) auch an die Methanolsyntheseanlage (5) angeschlossen ist.

5. Gasturbinen- und Dampfkraftwerk nach Anspruch 3, dadurch gekennzeichnet, daß ein Teil des die Wärmetauscheranlage (17) verlassenden Abgases des Eisenbadvergasers (10) über eine zweite Abgasleitung (43) direkt zur Methanolsyntheseanlage (5) leitbar ist.

6. Gasturbinen- und Dampfkraftwerk nach Anspruch 1, dadurch gekennzeichnet, daß eine Mitteldruckdampfleitung (19) in der in die Konvertierungsanlage (18) führende Abgasleitung (16) des Eisenbadvergasers (10) mündet.

7. Gasturbinen- und Dampfkraftwerk nach Anspruch 1, dadurch gekennzeichnet, daß ein Teil des die Wärmetauscheranlage (17) verlassenen Abgases des Eisenbadvergasers (10) über eine dritte Abgasleitung (44) direkt in die zur Brennkammer (27) der Gasturbine (28) führende Brennstoffleitung (29) leitbar ist.

8. Gasturbinen- und Dampfkraftwerk nach Anspruch 1, dadurch gekennzeichnet, daß die Methanolsyntheseanlage als Anlage (5) zur Durchlaufmethanolsynthese ausgebildet ist.

9. Gasturbinen- und Dampfkraftwerk nach Anspruch 1, dadurch gekennzeichnet, daß das in der der Konvertierungsanlage (18) nachgeschalteten Kohlendioxydwaschanlage (21) abgetrennte Kohlendioxyd über eine separate Leitung (45) in die Methanolsyntheseanlage einspeisbar ist.

10. Gasturbinen- und Dampfkraftwerk nach Anspruch 2, dadurch gekennzeichnet, daß dem Eisenbadvergaser (10) zusätzlich heizwertarme Brennstoffe, wie z. B. Braunkohle, Ölschiefer, Schwerölrückstände, Ölsand sowie deren Gemische, über die Kohleleitung (11) zuführbar sind.

11. Gasturbinen-und Dampfkraftwerk nach Anspruch 1, dadurch gekennzeichnet, daß der Eisenbadvergaser (10) an die Sauerstoffleitung (14) der Luftzerlegungsanlage (13) angeschlossen ist.

**Claims**

1. Gas turbine and steam power station with an integrated coal gasification plant — supplied, via an air separation plant, with oxygen — with a methanol synthesis plant — connected to the coal gasification plant with a steam power station part — connected to the waste heat boiler of the gas turbine — as well as with a nitrogen pipe — leading from an air separation plant to the combustion chamber of the gas turbine — characterised in that, the coal gasifier (8) is connected, for hydrogenating coal gasification, to a hydrogen supply pipe (7), an iron bath gasifier (10) is connected, downstream, to the coal gasifier, for the utilization of the residual coke, to which a conversion plant (18) and a carbon dioxide washing plant (21) - connected to the hydrogen supply pipe - are connected, downstream, on the exhaust gas side and, the exhaust gas pipe (22) of the coal gasifier is connected via a gas purification plant (24) to a methanol synthesis plant (5) and the exhaust gas pipe of the methanol synthesis plant (5) is connected to the combustion chamber (27) of the gas turbine (28).

2. Gas turbine and steam power station according to claim 1, characterised in that, the iron bath gasifier (10) can be supplied with, besides the residual coke of the coal gasifier (8), additional fossil fuels.

3. Gas turbine and steam power station according to claim 1, characterised in that, the exhaust gas pipe (16) of the iron bath gasifier is led through a heat exchanger plant (17) - connected, on the steam side, to the steam turbine (35) of the steam power station part (3).

4. Gas turbine and steam power station according to claim 1, characterised in that, the hydrogen supply pipe (7) is also connected to the methanol synthesis plant (5), as well as to the coal gasifier (8).

5. Gas turbine and steam power station according to claim 3, characterised in that, a part of the-exhaust gas of the iron bath gasifier (10) - leaving the heat exchanger plant (17) - can be piped, via a second exhaust gas pipe (43), directly to the methanol synthesis plant (5).

6. Gas turbine and steam power station according to claim 1, characterised in that, a medium pressure steam pipe (19) opens into the exhaust gas pipe (16) of the iron bath gasifier (10) - leading into the conversion plant (18).

7. Gas turbine and steam power station according to claim 1, characterised in that, a part of the exhaust gas of the iron bath gasifier (10) - leaving the heat exchanger plant (17) - can be piped, via a third exhaust gas pipe (44), directly into the fuel pipe (29) leading to the combustion chamber (27) of the gas turbine (28).

8. Gas turbine and steam power station according to claim 1, characterised in that, the methanol synthesis plant is formed as an installation (5) for continuous methanol synthesis.

9. Gas turbine and steam power station according to claim 1, characterised in that, the carbon dioxide, separated in the carbon dioxide washing plant (21) - connected, downstream, to the conversion plant (18) - can be fed, via a separate-pipe (45), into the methanol synthesis plant.

10. Gas turbine and steam power station according to claim 2, characterised in that, the iron bath gasifier (10) can be supplied in addition with fuels, poor in thermal value, such as eg. brown coal, oil shale, heavy oil tailings, oil sand and their mixes, via the coal pipe (11).

11. Gas turbine and steam power station according to claim 1, characterised in that, the iron bath gasifier (10) is connected to the oxygen pipe (14) of the air separation plant (13).

**Revendications**

1. Centrale à turbine à gaz et à vapeur comportant une installation intégrée de gazéification du charbon, alimentée en oxygène par l'intermédiaire d'une installation de fractionnement d'air, une installation de synthèse du méthanol raccordée à l'installation de gazéification du charbon, une partie formant centrale a vapeur raccordée à la chaudière de récupération de la chaleur perdue de la turbine à gaz ainsi qu'une canalisation d'alimentation en oxygène reliant l'installation de fractionnement d'air à la chambre de combustion de la turbine à gaz, caractérisée par le fait que le dispositif (8) de gazéification du charbon est raccordé, pour réaliser la gazéification du charbon avec hydrogénation, à une canalisation (7) d'alimentation en hydrogène, qu'un dispositif (10) de gazéification à bain de fer est branché en aval du dispositif de gazéification du charbon de manière à utiliser le coke résiduel, qu'une installation de conversion (18) et une installation (21) d'élimination du gaz carbonique par lavage, raccordée à la canalisation d'alimentation en hydrogène, sont branchées en aval sur le côté des gaz d'échappement et que la canalisation (22) des gaz d'échappement du dispositif de gazéification du charbon est raccordée par l'intermédiaire d'une installation (24) d'épuration des gaz à une installation (5) de synthèse du méthanol et que la canalisation des gaz d'échappement de l'installation (5) de synthèse du méthanol est raccordé à la chambre de combustion (27) de la turbine à gaz (28).

2. Centrale à turbine à gaz et à vapeur suivant la revendication 1, caractérisée par le fait que des combustibles fossiles supplémentaires peuvent être envoyés, en dehors du coke résiduel du dispositif (8) de gazéification du charbon, au dispositif (10) de gazéification à bain de fer.

3. Centrale à turbine à gaz et à vapeur suivant la revendication 1, caractérisée par le fait que la canalisation des gaz d'échappement (16) du dispositif de gazéification à bain de fer traverse une installation d'échange thermique (17) raccordée côté vapeur à la turbine à vapeur (35) de la partie (3) de la centrale à vapeur.

4. Centrale à turbine à gaz et à vapeur suivant la revendication 1, caractérisée par le fait que la canalisation (7) d'alimentation en hydrogène est raccordée non seulement au dispositif (8) de gazéification du charbon, mais également à l'installation (5) de synthèse du méthanol.

5. Centrale à turbine à gaz et à vapeur suivant la revendication 3, caractérisée par le fait qu'une partie des gaz d'échappement, qui quittent l'installation d'échange thermique (17), du dispositif (10) de gazéification à bain de fer peut être dirigée directement à l'installation (5) de synthèse du méthanol, par l'intermédiaire d'une seconde canalisation (43) des gaz d'échappement.

6. Centrale à turbine à gaz et à vapeur suivant la revendication 1, caractérisée par le fait qu'une canalisation de vapeur à moyenne pression (19) débouche dans la canalisation des gaz d'échappement (16) du dispositif (10) de gazéification à bain de fer, qui aboutit à l'installation de conversion (18).

7. Centrale à turbine à gaz et à vapeur suivant la revendication 1, caractérisée par le fait qu'une telle partie des gaz d'échappement, qui quitte l'installation d'échange thermique (17), du dispositif (10) de gazéification à bain de fer peut être envoyée directement, par l'intermédiaire d'une troisième canalisation des gaz d'échappement (44), dans la canalisation d'alimentation en combustible (29) aboutissant à la chambre de combustion (27) de la turbine à gaz (28).

8. Centrale à turbine à gaz et à vapeur suivant la revendication 1, caractérisée par le fait que l'installation de synthèse du méthanol est réalisée sous la forme d'une installation (5) servant à réaliser la synthèse continue du méthanol.

9. Centrale à turbine à gaz et à vapeur suivant la revendication 1, caractérisée par le fait que le gaz carbonique, qui est séparé dans l'installation (21) de lavage du gaz carbonique branchée en aval de l'installation de conversion (18), peut être produit dans l'installation de synthèse du méthanol.

10. Centrale à turbine à gaz et à vapeur suivant la revendication 2, caractérisée par le fait que des combustibles de haute valeur calorique, comme par exemple du lignite, du schiste bitumineux, des résidus d'huiles lourdes, du sable pétrolifère ainsi que des mélanges de ces produits, peuvent être envoyés en supplément au dispositif (10) de dégazage à bain de fer, par l'intermédiaire de la canalisation (11) d'alimentation en charbon.

11. Centrale à turbine à gaz et à vapeur suivant la revendication 1, caractérisée par le fait que le dispositif (10) de gazéification à bain de fer est raccordé à la canalisation d'alimentation en oxygène (14) de l'installation de fractionnement d'air (13).